(19) Europäisches Patentamt
European Patent Office
Office européen des brevets

(11) **EP 1 889 588 B1**

(12) **EUROPÄISCHE PATENTSCHRIFT**

(45) Veröffentlichungstag und Bekanntmachung des Hinweises auf die Patenterteilung:
**29.06.2011 Patentblatt 2011/26**

(51) Int Cl.:
***A61F 9/009*** *(2006.01)*

(21) Anmeldenummer: **06013828.6**

(22) Anmeldetag: **04.07.2006**

(54) **Verbessertes Augenkontaktelement**

Improved contact lens

Verre de contact amélioré

(84) Benannte Vertragsstaaten:
**DE ES FR GB IT**

(43) Veröffentlichungstag der Anmeldung:
**20.02.2008 Patentblatt 2008/08**

(73) Patentinhaber: **WaveLight GmbH**
**91058 Erlangen (DE)**

(72) Erfinder:
• **Triebel, Peter, Dr.**
**07751 Jena (DE)**
• **Kittelmann, Olaf, Dr.**
**14163 Berlin (DE)**
• **Vogler, Klaus, Dr.**
**90542 Eckental (DE)**

(74) Vertreter: **von Hellfeld, Axel**
**Wuesthoff & Wuesthoff**
**Patent- und Rechtsanwälte**
**Schweigerstrasse 2**
**81541 München (DE)**

(56) Entgegenhaltungen:
**WO-A-03/002008 US-A- 5 549 632**
**US-A1- 2002 103 481 US-A1- 2004 070 761**
**US-B1- 6 325 792**

**Beschreibung**

Die Erfindung betrifft eine verbesserte Applanationslinse oder Applanationsplatte für eine ophthalmologische Operation.

Gepulste Laserstrahlung wird in der Augenchirurgie beispielsweise zum Anbringen von Schnitten in der Hornhaut (Kornea) oder zum Abtrag (Ablation) von Gewebe aus der Hornhaut verwendet. Die eingestrahlte Laserstrahlung bewirkt im Hornhautgewebe einen photodisruptiven Prozess, der zur Gewebetrennung bzw. zur Entfernung von Gewebematerial führt. Derartige Bearbeitungen der Kornea finden beispielsweise im Rahmen von refraktiven Verfahren zur Minderung oder vollständigen Behebung von Fehlsichtigkeiten des Auges statt, bei denen die Kornea neu geformt wird und hierdurch ihre Brechungseigenschaften verändert werden.

Das dominierende refraktive Verfahren der Hornhautchirurgie ist die sogenannte LASIK (Laser in situ Keratomileusis). Hierbei wird aus der Hornhaut entweder mechanisch (mittels einer oszillierenden Schneidklinge in einem sogenannten Mikrokeratom) oder optisch (mittels Laserstrahlung, z.B. sogenannter Femtosekunden-Lasersysteme) ein kleiner Deckel herausgeschnitten, der mit einem Teil seines Randes noch an der Hornhaut hängt. Anschließend wird dieser üblicherweise auch als Flap bezeichnete Deckel zur Seite geklappt, wodurch das darunter liegende Stroma zugänglich wird. Mit Laserstrahlung wird dann nach Maßgabe eines für den jeweiligen Patienten ermittelten Ablationsprofils Stromagewebe abgetragen. Der Deckel wird danach wieder zurückgeklappt, wodurch die Wunde relativ schnell verheilen kann und die verbesserte Sehleistung innerhalb kürzester Zeit erreicht ist.

Ein Femtosekunden-Lasermikrokeratom umfasst eine Femtosekunden-Laserquelle, einen Scanner, der den Laserstrahl der Femtosekunden-Laserquelle sukzessive über einem Behandlungsbereich ablenkt, eine Fokussieroptik und eine Applanationsplatte oder Applanationslinse, die an der Hornhaut des Auges angeordnet ist. Ein derartiges System ist beispielsweise in der US 5,549,632 beschrieben.

Beim Einsatz eines Femtosekunden-Mikrokeratoms wird der LASIK-Schnitt in der Hornhaut durch eine nahezu flächenhafte Aneinanderreihung einer Mehrzahl von Fotomikrodisruptionen im Stroma der Hornhaut erzeugt. Die Fotomikrodisruptionen werden von Femtosekunden-Laserimpulsen erzeugt, die durch sehr hohe Intensitäten ($I > 10^{11}$ W/cm$^2$) eines Femtosekunden-Laserstrahles, der von einer Femtosekunden-Laserquelle erzeugt wird, entstehen und von einem geeignet dimensionierten optischen Strahlengang mit Umlenkspiegeln, einem Aufweitungsteleskop, einem Hochgeschwindigkeitsscanner und einem hochpräzisen, kurzbrennweitigen Fokussierobjektiv mit einer ausreichend hohen numerischen Apertur (NA > 0,20) zur Hornhaut geführt werden.

Um einen präzisen LASIK-Schnitt mit diesen Femtosekunden-Impulsen zu erreichen, muss die räumliche Lage eines Fokusbereiches des Femtosekunden-Impulses mit einer Genauigkeit von etwa 5 $\mu$m in allen drei Raumrichtungen im Gewebe der Kornea bestimmt sein. Auch die Größe des Fokusbereiches und die Lage der Fokusbereiche der aufeinanderfolgenden Impulse der Femtosekunden-Laserstrahlung müssen innerhalb einer Genauigkeit mit der gleichen Größenordnung, d.h. etwa 5$\mu$m, die vorgegebenen Werte und Positionen erreichen, um einen sicheren und qualitativ hochwertigen LASIK-Schnitt mit einem Femtosekunden-Lasersystem zu erreichen.

Für einen guten Behandlungserfolg ist ein möglichst kleiner Durchmesser d des Fokusbereiches erforderlich, um mit einer möglichst niedrigen Laserenergie E bei einer festgelegten Fluenz, d. h. Energiedichte, F (F = E/A) einen sicheren laserinduzierten optischen Durchbruch (LIOB; Laser Induced Optical Breakdown) zu erreichen. Hierbei wird der Schwellenwert $F_{th}$ für einen laserinduzierten optischen Durchbruch bereits bei einer niedrigen Laserimpulsenergie sicher überschritten. Dadurch kann eine Schädigung der Netzhaut und der Iris durch zu energiereiche und leistungsstarke Femtosekunden-Laserimpulse vermieden werden.

Für einen laserinduzierten optischen Durchbruch ist eine Fluenz von etwa 2 J/cm$^2$ bis etwa 3 J/cm$^2$ erforderlich. Kleine, eng benachbarte, präzise in der gleichen Tiefe lokalisierte Fotomikrodisruptionen (Durchmesser des Fokusbereiches $d_f$) stellen zudem die beste Schnittqualität, d.h. die niedrigste Rauhigkeit, bei dem Femtosekunden-LASIK-Verfahren bereit. Dabei ist die Überschreitung des LIOB-Schwellenwertes notwendig:

$$F = \frac{E}{A} = \frac{E}{0{,}25 \;\; \pi \;\; d_F{}^2} \geq F_{th} \;\; \approx \;\; 2...3 \;\; J/cm^2 \;\; .$$

Es ist zu erkennen, dass die Fluenz umgekehrt proportional zum Quadrat des Fokusdurchmessers ist, und folglich wird bei einem kleineren Durchmesser des Fokusbereiches die Fluenz auch bei einer niedrigen Laserimpulsenergie E höher als der Schwellenwert $F_{th}$ für einen laserinduzierten optischen Durchbruch sein.

Theoretisch kann ein Femtosekunden-Laserimpuls bestenfalls auf einen Wert in der Größenordnung des Durchmessers $d_A$ der Airy-Funktion fokussiert werden. Es gilt

$$d_A \approx 2{,}44 \frac{\lambda}{D} f \; ;$$

woraus bestenfalls ein minimaler Fokusdurchmesser $d_F \approx d_A$ folgt:

$$d_A \approx d_F \approx 2{,}44 \frac{\lambda}{D} f \; ;$$

wobei f die Brennweite des Fokussierobjektives, $\lambda$ die Wellenlänge der Femtosekunden-Laserstrahlung und D die Apertur oder der Durchmesser des Laserstrahles auf der Fokussierlinse ist.

Dies setzt allerdings einen nahezu perfekten Laserstrahl (im Grundmode bzw. einer ebenen Welle) und eine beugungsbegrenzte Fokussierung durch ein aberationsfreies Objektiv der Brennweite f voraus.

Daher müssen hohe Anforderungen an die optische Qualität der Bauelemente des gesamten optischen Strahlenganges, den die Femtosekunden-Laserstrahlung durchläuft, gestellt werden. Neben einer hohen Gesamttransmission, die den Energieverlust der Femtosekunden-Impulse auf dem Weg zum Behandlungsort, d. h. dem Auge bzw. der Kornea, minimiert, stellt dies insbesondere hohe Anforderungen an die Aberationsfreiheit der verwendeten optischen Bauteile. Zusätzlich ist eine möglichst niedrige Deformation der Wellenfront der Laserstrahlung erforderlich. Diese wird typischerweise durch die Ebenheit, die Homogenität und die verzerrungsfreie optische Führung des Femtosekunden-Laserstrahles in Form von Bruchteilen der Wellenlänge ausgedrückt, beispielsweise $\lambda/n$. Es versteht sich, dass die kostspieligsten und aufwändigsten optischen Bauteile des Femtosekunden-Laserstrahlenganges, beispielsweise das Aufweitungsteleskop und das Fokussierobjektiv, mit dieser hohen Aberationsfreiheit spezifiziert werden. Aber auch die im Strahlengang verwendeten Umlenkspiegel sowie die im Scanner eingesetzten Ablenkspiegel müssen die Anforderungen an eine hohe Ebenheit und eine geringe Deformation der Wellenfront des Femtosekunden-Laserimpulses erfüllen.

Eine von einem beliebigen optischen Element deformierte Wellenfront lässt sich nicht ohne weiteres durch ein anderes optisches Element korrigieren und verhindert die gewünschte bestmögliche, d. h. "scharfe", Fokussierung, die im Fall einer deformierten Wellenfront auch nicht mit einer qualitativ hochwertigen Fokussieroptik erreicht werden kann.

Bei dem Femtosekunden-LASIK-Verfahren werden üblicherweise als Schnittstelle mit dem Auge des Patienten sogenannte Saugringhalterungen verwendet, die mittels eines Unterdruckes an das Auge des Patienten angesaugt werden. Dadurch wird das Auge mit einer Vorrichtung gekoppelt, die ein Kontaktglas, beispielsweise eine sogenannte Applationsplatte bzw. Applanationslinse, umfasst, das die Kornea berührt. Dadurch befindet sich das Auge in einer definierten Position zum Fokussierobjektiv des Femtosekunden-Laserstrahles.

Ferner ist zu beachten, dass das Kontaktglas eine Referenzebene bildet, zu der die Fokuslage des Femtosekunden-Laserstrahles orientiert werden kann. Diese Orientierung ist speziell für die Z-Richtung, d. h. für die Lage der Fokustiefe jenseits des Kontaktglases in der Kornea, wichtig, um einen LASIK-Schnitt genau in der gewünschten Tiefe, beispielsweise etwa 120 $\mu$m mit einer entsprechenden Tiefengenauigkeit von weniger als $\pm$ 10 $\mu$m durchführen zu können, wie beispielsweise in der US 6,899,707 B2 beschrieben ist.

Das verwendete Kontaktglas kann sphärisch oder plan ausgebildet sein. Ein als planare Applanationsplatte ausgebildetes Kontaktglas erleichtert die Aufrechterhaltung einer einheitlichen Fokustiefe des Femtosekunden-Laserstrahles, aber sie erhöht durch die Applanation der Hornhautkrümmung den Augendruck deutlich stärker, d. h. um mehr als etwa 100 mm Hg (0,133 bar), als ein als sphärisch gekrümmte Applanationslinse ausgebildetes Kontaktglas, die die natürliche Krümmung der Hornhaut mehr oder weniger gut nachbildet, was allerdings einen höheren Aufwand für die Steuerung der einheitlichen Fokustiefe, beispielsweise durch eine schnelle Verschiebung der Brennweite des Fokussierobjektives in der Z-Achse, nach sich zieht.

Die US 6,899,707 B2 beschreibt eine Applanationslinse mit einer Transmission von mehr als 90% in einem Wellenlängenbereich von 275 nm bis 2500 nm. Die US 6,730,074 B2 schlägt eine Kontaktlinse vor, deren Krümmung der Hornhautkrümmung entspricht. Während der LASIK-Behandlung wird der Fokuspunkt in der Z-Richtung verschoben, um die Krümmungseffekte zu kompensieren. Die US 6,342,053 B1 schlägt eine an das Auge eines Patienten gekoppelte lichtdurchlässige Formgebungseinrichtung vor. Der Krümmungsradius der lichtdurchlässigen Formgebungseinrichtung entspricht etwa der gewünschten emmetropischen Form des vorderen Bereiches der Kornea. Beispielsweise wird Infrarotstrahlung, auf Gewebe in der Kornea gewichkt, um das Gewebe bei ausreichender Temperatur zum Schnumpfen zu bringen .Dadurch wird die Kornea neu geformt, und die neue Form der Kornea entspricht der Krümmung des dem Auge zugewandten Bereiches der lichtdurchlässigen Formgebungseinrichtung.

Die US 2002/0103481 A1 offenbart, dass es bei ophtalmologlschen Operationen wichtig ist, dass die Fokuspunkte den korrekten Punktdurchmesser und die korrekte Form aufweisen. Dazu muss der Laserstrahl möglichst frei von Aberrationen sein. Insbesondere bei ophtalmologischen Laseroperationen, die die Kornea betreffen, führt die sphärische Geometrie der Kornea optische Aberrationen ein, die von den Aberrationen, die durch das optische System des Lasers verursacht werden, separat sind und sich von diesen unterscheiden. Diese durch die Kornea verursachten Aberrationen stören signifikant die Definition der Fokuspunkte eines Laserstrahls, während der Strahl auf eine Position innerhalb des Korneagewebes fokussiert wird. Aufgrund der sphärischen Geometrie der vorderen Oberfläche der Kornea treten eine sphärische Aberration und ein Koma auf. Es wird vorgeschlagen, dass eine applanare Brechung der vorderen Oberfläche der Kornea effektiv durch Abflachen der vorderen Oberfläche erreicht werden kann. Bei einer derartigen Korneakonfiguration weist der Strahl keine Aberrationen auf, die andernfalls am Übergang in die ursprüngliche sphärische vordere Oberfläche der Kornea auftreten würde. Es wird ferner vorgeschlagen, zum Reduzieren der Aberrationen der Kornea die Korneaoberfläche mittels einer Applanationslinse abzuflachen.

Weitere Beispiele für Kontaktgläser, d. h. Applanationsplatten oder Applanationslinsen, finden sich in der EP 1 034 755 A1, der EP 1 034 757 A, der US 6,623,476 B2, der US 6,999,707 B2, der US 5,549,632, der US 6 325 792 und der WO 2005/079717 A1.

Es ist eine Aufgabe der Erfindung, ein verbessertes Kontaktelement für das Auge eines Patienten zu schaffen.

Die Aufgabe wird durch ein optisches Augenkontaktelement nach Anspruch 1 gelöst, das zumindest teilweise lichtdurchlässig ist und dazu ausgebildet ist, dass es im Einsatz an der Kornea anliegt, wobei das optische Augenkontaktelement einen Wellenfrontfehler von maximal etwa $\lambda/2$, vorzugsweise von maximal etwa $\lambda/4$, höchst vorzugsweise von maximal etwa $\lambda/10$, in einem Wellenlängenbereich 7 des durchlaufenden Lichtstrahls (3) von etwa 1000nm bis etwa 1200 nm verursacht, wobei das optische Angenkontaktelement (48) einen Brechengsinder von etwa 1,35 bis etwa 1,4 aufweist. Das optische Augenkontaktelement kann eine sogenannte Applanationsplatte oder Applanationslinse sein.

Um einen sicheren Schnitt mit einem Femtosekunden-Lasermikrokeratom zu erreichen, werden hohe Anforderungen an die Strahlqualität einer Femtosekunden-Laserquelle, einer Fokussieroptik und einer Aufweitungsoptik gestellt, die die Femtosekunden-Laserstrahlung durchläuft. Allerdings hat der Fachmann bisher das letzte, aber nicht unwesentliche Element, d. h. das Augenkontaktelement, im optischen Strahlengang bisher nicht in die optische Qualitätsbetrachtung einbezogen. Es versteht sich, dass dieses relativ einfache Element die zuvor mit aufwändigen Mitteln aufrecht erhaltene Wellenfrontqualität beim Durchlauf des Femtosekunden-Laserimpulses noch derart verschlechtern kann, dass die Fokussierbarkeit der Femtosekunden-Laserstrahlung erheblich darunter leidet, und unter Umständen kein laserinduzierter optischer Durchbruch und/oder kein Plasma in der Kornea entsteht, wobei folglich der LASIK-Schnitt nicht oder nur in einer schlechteren Qualität gelingt bzw. mit einer erheblich höheren Femtosekunden-Impulsenergie erzeugt werden muss.

Das optische Kontaktelement verursacht den wellenfrontfehler von maximal etwa $\lambda/2$, vorzugsweise maximal etwa $\lambda/4$, höchst vorzugsweise von maximal etwa $\lambda/10$, in einem Wellenlängenbereich des dadurch durchlaufenden Lichtstrahls von etwa 1000 nm bis etwa 1200 nm. Eine typische Femtosekunden-Laserquelle erzeugt Laserimpulse, beispielsweise mit einer Wellenlänge von etwa 1035 nm $\pm$ 10 nm. Zumindest in diesem Bereich muss bei einer Ausführungsform das optische Augenkontaktelement den niedrigen Wellenfrontfehler aufweisen, wobei sich bei einer Wellenlänge von etwa 520 nm etwa der doppelte Wellenfrontfehler ergeben kann.

Das optische Augenkontaktelement hat einen Brechungsindex $\eta_1$ von etwa 1,35 bis etwa 1,40, vorzugsweise von etwa 1,36 bis 1,38, höchst vorzugsweise von etwa 1,37 aufweisen. Der Brechungsindex $\eta_2$ der Kornea beträgt etwa 1,37, und falls der Brechungsindex des optischen Augenkontaktelementes einen ähnlichen Brechungsindex aufweist, wird die Qualität und/oder die Intensität eines Lichtstrahles bzw. eines Laserstrahles beim Übergang vom optischen Augenkontaktelement in die Kornea nicht verringert.

Die Reflektionsverluste R werden wie folgt berechnet:

$$R = \left(\frac{\eta_2 - \eta_1}{\eta_2 + \eta_1}\right)^2;$$

Bei $\eta_2 \approx \eta_1$ ergibt sich, dass nahezu keine Reflektionsverluste auftreten.

Das optische Augenkontaktelement kann biokompatibel sein. Biokompatible Materialien weisen keinen negativen Einfluss auf das Auge auf. Das optische Augenkontaktelement kann eine biokompatible Schicht an dem Bereich aufweisen, der im Einsatz das Auge berührt. Die biokompatible Schicht kann beispielsweise Proteine aufweisen.

Das optische Kontaktelement kann eine hohe Stabilität gegenüber Femtosekunden-Laserimpulsen aufweisen. Dies ist insbesondere wegen der hohen Energiedichte der Laserimpulse wichtig. Die hohe Stabilität gegenüber hohen Strahlungsintensitäten (hohe Damage-Schwelle), beispielsweise gegenüber Femtosekunden-Laserimpulsen, kann beispielsweise durch eine hohe Transmission des optischen Augenkontaktelementes erreicht werden. Das optische Augenkontaktelement kann beispielsweise Glas vom Typ BK7 aufweisen. Ein Glas vom Typ BK7 mit einer Dicke von 10 mm kann in einem Wellenlängenbereich von etwa 370 nm bis etwa 1700 nm eine Transmission von mehr als etwa 90% aufweisen, wobei sich bei einer niedrigeren Dicke des Glases eine höhere Transmission ergibt. Das optische Augenkontaktelement kann auch Quarzglas (fused silica) aufweisen.

Das optische Augenkontaktelement kann einen optischen Kunststoff aufweisen. Dadurch wird das optische Augenkontaktelement trotz hoher Qualität relativ kostengünstig.

Ein weiterer Aspekt der Erfindung betrifft ein Femtosekunden-Lasersystem, das eine Femtosekunden-Laserquelle und das vorstehend beschriebene Augenkontaktelement umfasst. Das Femtosekunden-Lasersystem kann ferner einen Scanner mit zumindest einem Ablenkspiegel zum Positionieren des Femtosekunden-Laserstrahles auf einem Behandlungsort auf dem Auge eines Patienten und eine Fokussieroptik zum Fokussieren des Femtosekunden-Laserstrahles umfassen.

Die Erfindung wird jetzt unter Bezugnahme auf die begleitenden Zeichnungen detaillierter beschrieben, wobei

Fig. 1 eine schematische, stark vereinfachte Ansicht eines Femtosekunden-Mikrokeratoms ist,

Fig. 2 die Lage und den Durchmesser der Fokusbereiche bei einem herkömmlichen optischen Augenkontaktelement zeigt, und

Fig. 3 die Lage und den Durchmesser der Fokusbereiche bei einem erfindungsgemäßen optischen Augenkontaktelement zeigt.

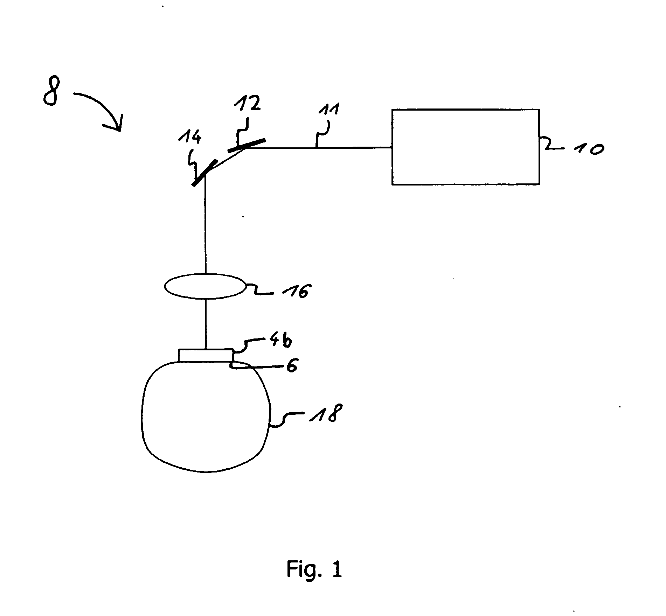

Fig. 1 zeigt ein Femtosekunden-Mikrokeratom mit einer Femtosekunden-Laserquelle 10, die einen Femtosekunden-Laserstrahl 11 mit einem niedrigen Wellenfrontfehler erzeugt. Der Femtosekunden-Laserstrahl wird durch einen ersten Ablenkspiegel 12 und einen zweiten Ablenkspiegel 14 eines optischen Scanners abgelenkt, so dass ein beliebiger Punkt im Behandlungsbereich auf der Kornea 6 eines Patientenauges 18 erreicht werden kann. Der von dem ersten Ablenkspiegel 12 und dem zweiten Ablenkspiegel 14 abgelenkte Femtosekunden-Laserstrahl 11 wird von einer Fokussieroptik 16 fokussiert und tritt in ein erfindungsgemäßes optisches Kontaktelement 4b ein. Das erfindungsgemäße optische Augenkontaktelement 4b applaniert die Kornea 6. Dadurch kann ein definierter Abstand zwischen der Fokussieroptik 16 und der Kornea 6 eingehalten werden. Beim Austritt des Femtosekunden-Laserstrahles 11 aus dem optischen Kontaktelement entsteht etwa im Fokusbereich des Femtosekunden-Laserstrahles 11, d.h. etwa in der Ebene der Brennweite des Fokussierobjektives 16, ein laserinduzierter optischer Durchbruch. Indem eine Mehrzahl von Femtosekunden-Laserstrahlen 11 sukzessive über den Behandlungsbereich in der Kornea 6 gelenkt werden, entsteht ein flächenhafter Schnitt im Inneren der Kornea 6 des Auges 18.

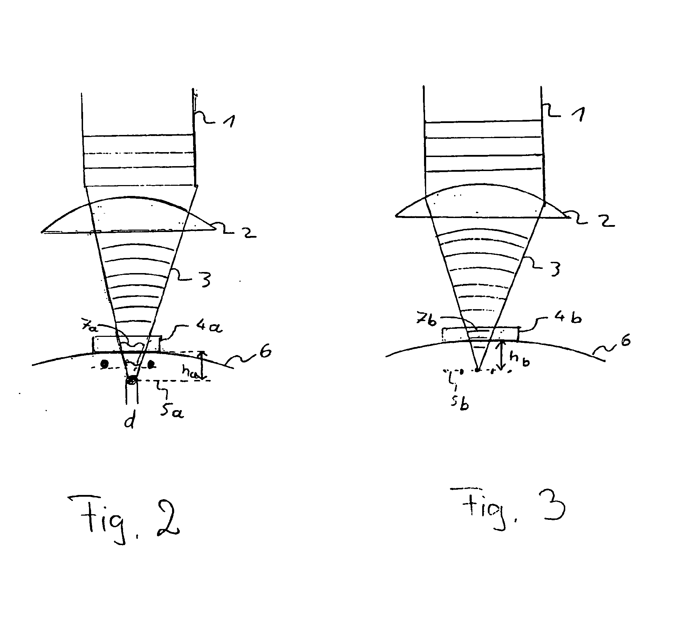

Fig. 2 zeigt den Wellenverlauf bei einem herkömmlichen Augenkontaktelement 4a. Ein Femtosekunden-Laserstrahl 1 von sehr hoher Qualität wird auf eine Fokussierlinse 2 guter Qualität, die beispielsweise einen Wellenfrontfehler von $\lambda/10$ aufweist, gerichtet. Die Fokussierlinse 2 bündelt den einfallenden Femtosekunden-Laserstrahl 1 in einen fokussierten Femtosekunden-Laserstrahl 3, der immer noch eine hohe Qualität aufweist. Im Kontext dieser Erfindung bedeutet hohe Qualität eines Laserstrahles einen geringen Wellenfrontfehler. Der fokussierte Femtosekunden-Laserstrahl trifft auf ein herkömmliches Augenkontaktelement 4a, beispielsweise eine Applanationsplatte oder Applanationslinse, auf. Herkömmliche Augenkontaktelemente verursachen beispielsweise einen Wellenfrontfehler von $2,2\lambda$. Aufgrund der niedrigen optischen Qualität des herkömmlichen Augenkontaktelementes entsteht ein Wellenfrontfehler 7a. Der Durchmesser der aus dem fokussierten Femtosekunden-Laserstrahl 3 entstehenden Fokusbereiche 5a ist daher wesentlich größer als der aufgrund der Airy-Funktion erreichbare theoretische Durchmesser. Ferner befinden sich die Fokusbereiche 5a aufgrund der in dem herkömmlichen Augenkontaktelement entstehenden Wellenfrontfehler auf unterschiedlichen und/oder uneinheitlichen Fokustiefen $h_a$.

Aufgrund des relativ großen Durchmessers der Fokusbereiche 5a ist eine höhere Laserimpulsenergie erforderlich, um den laserinduzierten optischen Durchbruch für einen Schnitt in der Kornea zu erreichen. Ferner wird nicht das optimale Behandlungsergebnis, d. h. Schnittqualität, erzielt, da sich die Fokusbereiche 5a in einer unterschiedlichen und/oder uneinheitlichen Tiefe $h_a$ befinden und daher ein Femtosekunden-Laserschnitt mit hoher Rauheit entsteht.

Fig. 3 zeigt einen Wellenfrontfehler bei einem erfindungsgemäßen optischen Augenkontaktelement. Fig. 3 ähnelt Fig. 2 und ähnliche Bauteile und Figurenelemente werden mit den gleichen Bezugszeichen gekennzeichnet.

Der Femtosekunden-Laserstrahl 1 von hoher Qualität, d. h. einem niedrigen Wellenfrontfehler, wird durch eine Fokussierlinse 2, die einen Wellenfrontfehler von etwa $\lambda/10$ verursacht, in einen fokussierten Femtosekunden-Laserstrahl 3 mit einem niedrigen Wellenfrontfehler gebündelt. Der fokussierte Femtosekunden-Laserstrahl 3 durchläuft ein optisches Augenkontaktelement 4b, das einen Wellenfrontfehler von maximal etwa $\lambda/2$, vorzugsweise maximal etwa $\lambda/4$, höchst vorzugsweise maximal etwa $\lambda/10$, verursacht. Aufgrund des niedrigen von dem erfindungsgemäßen optischen Augenkontaktelement 4b verursachten Wellenfrontfehlers, haben die Wellenfronten 7b weiterhin eine hohe Qualität. Die aus dem fokussierten Femtosekunden-Laserstrahl 3 entstehenden Fokusbereiche in der Kornea weisen daher nahezu den minimalen Durchmesser auf, der sich aus der Airy-Funktion ergibt. Ferner befinden sich die Fokusbereiche in einer nahezu konstanten Tiefe $h_b$ in der Kornea 6, und die Rauheit des Schnittes ist gering.

Simulationen ergaben, dass sich bei einem Femtosekunden-Laserstrahl mit einer Wellenlänge von 1035 nm ± 2,5 nm und einem herkömmlichen optischen Augenkontaktelement, das einen Wellenfrontfehler von 2,2λ verursacht, ein Radius des Fokusbereiches von ≥ 30 μm ergibt. Das Zentrum der Fokusbereiche, würde sich bei Luft in einer Entfernung von 220 μm von der Grenzfläche zwischen dem optischen Augenkontaktelement und der Luft befinden. Bei einem herkömmlichen optischen Augenkontaktelement entsteht ein Wellenfrontfehler PV (peak-valley) in der Fokusebene von 1,41 λ.

Unter den gleichen Bedingungen ergibt sich bei einem idealen optischen Augenkontaktelement, das einen Wellenfrontfehler von 0λ verursacht, ein Radius von ≤ 15 μm für den Fokusbereich. In Luft läge das Zentrum des Fokusbereiches in einem Abstand von 380 μm von der Grenzfläche zwischen dem optischen Augenkontaktelement und der Luft. Es ergibt sich ein Wellenfrontfehler PV des Laserstrahles von nur 0,62λ im Fokusbereich.

Bei der vorstehenden Simulation wies das erfindungsgemäße Augenkontaktelement 4b eine Dicke von 7 mm auf, und wurde aus einer planparallelen Platte mit dem Material BK7 ausgebildet. Der Eingangsstrahl hatte einen Durchmesser von 15 mm mit einer gauss-förmigen ebenen Welle. Das Bearbeitungsfeld hatte einen Durchmesser von 6 mm. Das Fokussierungsobjektiv umfasste zwei Zerstreuungslinsen und eine Fokussierungslinse. Es wurden keine Fertigungstoleranzen und keine Asphären des Fokussierungsobjektives berücksichtigt. Die Fokuslänge des Objektives in Luft betrug 38 mm ausgehend von der letzten Hauptebene.

Die Simulation stellt lediglich eine grobe Demonstration des Einflusses der Wellenfrontqualität des optischen Kontaktelementes dar. Bei realen Systemen mit einem präzisen Fokussierobjektiv, d.h. nicht einem einfachen Objektiv mit drei Linsen wie bei der vorliegenden einfachen Simulation, ist der Einfluss der durchschnittlichen Wellenfrontqualität des optischen Kontaktelementes deutlich höher, da mit den besten optischen Einrichtungen tatsächlich Fokusdurchmesser von $d_f \approx 5$ μm erreicht werden. Das Ergebnis des Einflusses einer nicht optimierten Applanationsplatte wäre mit einem Fokusdurchmesser von $d_f \gtrsim 30$ μm deutlich schlechter. Auch das in der Praxis größere Scanfeld von etwa 10 bis 12 mm erhöht die Unterschiede bei der Verwendung eines optischen Kontaktelementes mit einer guten Wellenfrontfehlerkorrektur gegenüber einem optischen Kontaktelement mit einer schlechten Wellenfrontfehlerkorrektur tendenziell stark.

Die Erfindung hat den Vorteil, dass der Durchmesser der Fokusbereiche nahezu den minimalen theoretisch möglichen Wert aufweist, wodurch lediglich eine niedrigere Femtosekunden-Impulsenergie zur Erzeugung eines laserinduzierten optischen Durchbruches erforderlich ist. Ferner ermöglicht das erfindungsgemäße optische Augenkontaktelement Schnitte von höherer Qualität, da sich der Mittelpunkt der Fokusbereiche in einem definierten Abstand vom optischen Augenkontaktelement befindet.

## Patentansprüche

1.   Optisches Augenkontaktelement (4b), das zumindest teilweise lichtdurchlässig ist und dazu ausgebildet ist, dass es im Einsatz an der Kornea anliegt, wobei das optische Augenkontaktelement (4b) einen Wellenfrontfehler von maximal etwa λ/2 in einem Wellen Längenbereich λ des durch laufen den Lichtstrahls (3) von etwa 1000 nm bis etwa 1200 nm verursacht, **dadurch gekennzeichnet dass**, das optische Augenkontaktelement (4b) einen Brechungsindex von etwa 1,35 bis etwa 1,40 aufweist.

2.   Optisches Augenkontaktelement (4b) nach Anspruch 1, **dadurch gekennzeichnet, dass** das optische Augenkontaktelement (4b) einen Wellenfrontfehler von maximal etwa λ/4 bei dem durchlaufenden Lichtstrahl (3) verursacht.

3.   Optisches Augenkontaktelement (4b) nach Anspruch 1 oder 2, **dadurch gekennzeichnet, dass** das optische Augenkontaktelement (4b) einen Wellenfrontfehler von maximal etwa λ/10 bei dem durchlaufenden Lichtstrahl (3) verursacht.

4.   Optisches Augenkontaktelement (4b) nach einem der vorstehenden Ansprüche, **dadurch gekennzeichnet, dass** das optische Augenkontaktelement (4b) biokompatibel ist.

5.   Optisches Augenkontaktelement (4b) nach einem der vorstehenden Ansprüche, **dadurch gekennzeichnet, dass** das optische Augenkontaktelement (4b) sterilisierbar ist.

6.   Optisches Augenkontaktelement (4b) nach einem der Ansprüche 1 bis 5, **dadurch gekennzeichnet, dass** das optische Augenkontaktelement (4b) einen optischen Kunststoff aufweist.

7.   Femtosekunden-Lasersystem (8), umfassend eine Femtosekunden-Laserquelle (10) und ein optisches Augenkontaktelement (4b) nach einem der Ansprüche 1 bis 6.

**EP 1 889 588 B1**

**Claims**

1. Optical eye-contact element (4b), which is at least partly translucent and which, in use, abuts on the cornea, wherein the optical eye-contact element (4b) gives rise to a wavefront error of at most about $\lambda/2$ in a wave length range of the passing light beam (3) from about 1000 nm to about 1200 nm, **characterized in that** the optical eye-contact element (4b) exhibits a refractive index from about 1.35 to about 1.40.

2. Optical eye-contact element (4b) according to claim 1, **characterized in that** the optical eye-contact element (4b) gives rise to a wavefront error of at most about $\lambda/4$ in a passing light beam (3).

3. Optical eye-contact element (4b) according to claim 1 or 2, **characterized in that** the optical eye-contact element (4b) gives rise to a wavefront error of at most about $\lambda/10$ in a passing light beam (3).

4. Optical eye-contact element (4b) according to one of the preceding claims, **characterized in that** the optical eye-contact element (4b) is biocompatible.

5. Optical eye-contact element (4b) according to one of the preceding claims, **characterized in that** the optical eye-contact element (4b) can be sterilized.

6. Optical eye-contact element (4b) according to one of the claims 1 to 5, **characterized in that** the optical eye-contact element (4b) comprises an optical plastic.

7. Femtosecond-laser system (8) including a femtosecond-laser source (10) and an optical eye-contact element (4b) according to one of the claims 1 to 6.


**Revendications**

1. Verre de contact (4b) laissant passer pour le moins partiellement la lumière et conçu pour être appliqué sur la cornée pendant son utilisation, ledit verre de contact (4b) engendrant une erreur de front d'onde d'environ $\lambda/2$ maximum dans une plage de longueur d'onde $\lambda$ du rayon lumineux traversant (3) située entre environ 1000 nm et environ 1200 nm, **caractérisé en ce que** le verre de contact (4b) présente un indice de réfraction compris entre environ 1,35 et environ 1,40.

2. Verre de contact (4b) selon la revendication 1, **caractérisé en ce que** le verre de contact (4b) engendre pour le rayon lumineux traversant (3) une erreur de front d'onde d'environ $\lambda/4$ maximum.

3. Verre de contact (4b) selon la revendication 1 ou 2, **caractérisé en ce que** le verre de contact (4b) engendre pour le rayon lumineux traversant (3) une erreur de front d'onde d'environ $\lambda/10$ maximum.

4. Verre de contact (4b) selon l'une des revendications précédentes, **caractérisé en ce que** le verre de contact (4b) est biocompatible.

5. Verre de contact (4b) selon l'une des revendications précédentes, **caractérisé en ce que** le verre de contact (4b) peut être stérilisé.

6. Verre de contact (4b) selon l'une des revendications 1 à 5, **caractérisé en ce que** le verre de contact (4b) présente une matière plastique optique.

7. Système laser femtoseconde (8), comprenant une source laser femtoseconde (10) et un verre de contact (4b) selon l'une des revendications 1 à 6.

**Fig. 1**

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5549632 A
- US 6899707 B2
- US 6730074 B2
- US 6342053 B1
- US 0103481 A1
- EP 1034755 A1
- EP 1034757 A
- US 6623476 B2
- US 6999707 B2
- US 6325792 B
- WO 079717 A1